(19) Europäisches Patentamt
European Patent Office
Office européen des brevets

(11) Publication number: **0 079 521 A1**

(12) **EUROPEAN PATENT APPLICATION**

(21) Application number: 82110114.4

(22) Date of filing: 03.11.82

(51) Int. Cl.[3]: C 07 C 103/52
C 07 C 102/00

(30) Priority: 09.11.81 US 319170
09.08.82 US 404994

(43) Date of publication of application: 25.05.83 Bulletin 83/21

(84) Designated Contracting States: CH DE FR GB IT LI NL

(71) Applicant: MERCK & CO. INC.
126, East Lincoln Avenue P.O. Box 2000
Rahway New Jersey 07065(US)

(72) Inventor: Greenlee, William J.
115 Herrick Avenue
Teaneck New Jersey 07666(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Process for preparation of carboxyalkyldipeptide derivatives.**

(57) A process for the preparation of carboxyalkyldipeptide compounds of the Formula

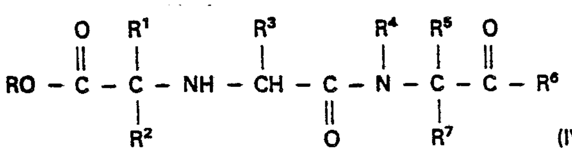

(IV)

is described wherein

$R^6$ is hydroxy, loweralkoxy, or amino;
R, $R^2$ and $R^7$ are hydrogen or loweralkyl;
$R^3$ is, e.g., hydrogen, or loweralkyl,
$R^1$ is, e.g., hydrogen, or alkyl of from 1 to 20 carbon atoms,
$R^4$ is hydrogen or loweralkyl,
$R^5$ is, e.g., hydrogen, or loweralkyl, and,
$R^4$ and $R^5$ may be connected together to form an alyklene bridge of from 2 to 4 carbon atoms.

The process comprises reacting a compound of the Formula

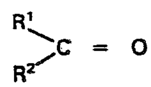

(I)

with a dipeptide (or protected dipeptide) of the Formula

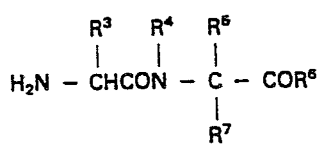

(II)

and an alkali metal cyanide or a trimethylsilycyanide, to form an amino nitrile compound of the Formula

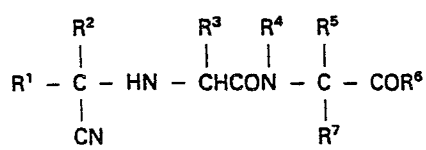

(III)

followed by hydrolysis.

These compounds are useful as converting enzyme inhibitors and as antihypertensive agents.

EP 0 079 521 A1

## TITLE OF THE INVENTION

PROCESS FOR PREPARATION OF CARBOXYALKYLDIPEPTIDE DERIVATIVES

## BACKGROUND OF THE INVENTION

The preparation and pharmaceutical use of carboxyalkyldipeptide derivatives (See Formula IV, below) have been described in Patchett et. al., European Patent Application No. 12,401 which is incorporated herein by reference. These compounds are useful as converting enzyme inhibitors and as antihypertensive agents.

## SUMMARY OF THE INVENTION

This invention relates to a process for the preparation of the carboxyalkyldipeptide compounds of Formula (IV) shown below.

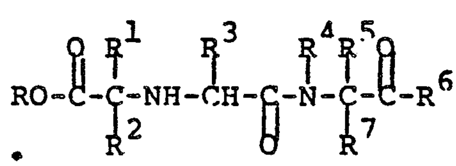

(IV)

DETAILED DESCRIPTION OF THE INVENTION

The present invention is a process for preparing carboxylalkyldipeptide compounds of Formula (IV) illustrated by the following equation:

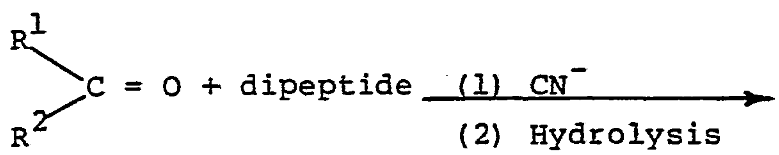


I II

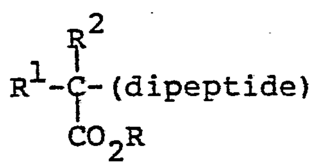

IV

wherein:

$R^1$ is hydrogen,

alkyl of from 1 to 20 carbon atoms which include branched and cyclic and unsaturated (such as allyl) alkyl groups,

substituted loweralkyl wherein the substituent can be halo, hydroxy, lower alkoxy, aryloxy such as phenoxy, amino, diloweralkylamino, acylamino, such as acetamido and benzamido, arylamino, guanidino, imidazolyl, indolyl, mercapto, loweralkylthio, arylthio such as phenylthio, carboxy or carboloweralkoxy,

aryl such as phenyl or naphthyl,

substituted aryl such as phenyl wherein the

substituent is lower alkyl, lower alkoxy or halo,

arloweralkyl, arloweralkenyl, heteroarloweralkyl or heteroarloweralkenyl such as benzyl, styryl or indolyl ethyl,

substituted arloweralkyl, substituted arloweralkenyl, substituted heteroarloweralkyl, or substituted heteroarloweralkenyl, wherein the substituent(s) is halo, dihalo, lower alkyl, hydroxy, lower alkoxy, amino, aminomethyl, acylamino (acetylamino or benzoylamino) diloweralkylamino, loweralkylamino, carboxyl, haloloweralkyl, or sulfonamido;

arloweralkyl or heteroarloweralkyl substituted on the alkyl portion by amino or acylamino (acetylamino or benzoylamino);

and

R and $R^2$ are hydrogen or loweralkyl;

and the dipeptide (II) has the formula:

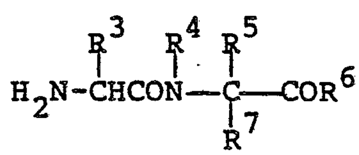

II

wherein:

$R^4$ and $R^7$ are hydrogen or loweralkyl;

$R^5$ is hydrogen, loweralkyl, phenyl, phenylloweralkyl, hydroxyphenylloweralkyl, hydroxy-

loweralkyl, aminoloweralkyl, guanidinoloweralkyl, imidazolylloweralkyl, indolylloweralkyl, mercaptoloweralkyl or loweralkylthioloweralkyl;

$R^4$ and $R^5$ may be connected together to form an alkylene bridge of from 2 to 4 carbon atoms, an alkylene bridge of from 2 to 3 carbon atoms and one sulfur atom, an alkylene bridge of from 2 to 4 carbon atoms containing a double bond or an alkylene bridge as above substituted with hydroxy, loweralkoxy, loweralkyl or diloweralkyl;

$R^3$ is hydrogen, loweralkyl, phenylloweralkyl, aminomethylphenylloweralkyl, hydroxyphenylloweralkyl, hydroxyloweralkyl, acylaminoloweralkyl (such as benzoylaminoloweralkyl, acetylaminoloweralkyl), aminoloweralkyl, dimethylaminoloweralkyl, haloloweralkyl, guanidinoloweralkyl, imidazolylloweralkyl, indolylloweralkyl, mercaptoloweralkyl, loweralkylthioloweralkyl;

and $R^6$ is hydroxy, lower alkoxy, or amino.

The loweralkyl or loweralkenyl groups represented by any of the variable R groups include straight and branched chain hydrocarbon radicals of from one to six carbon atoms, for example, methyl, ethyl, propyl, isopropyl, butyl, isobutyl, t-butyl, pentyl, isopentyl, hexyl or vinyl, allyl, butenyl and the like. The aralkyl groups represented by any of the above variable R groups have from one to four carbon atoms in the alkyl portion thereof and include

for example, benzyl, p-methoxybenzyl and the like. Halo means chloro, bromo, iodo or fluoro. Arvl where it appears in any of the radicals represents phenvl or naphthyl. Heteroaryl groups where they appear include for example pyridyl, thienyl, furyl, indolyl, benzthienyl, imidazoyl and thiazolyl.

The $R^1$, $R^3$ and $R^5$ substituted lower alkyl moieties are exemplified by groups such as

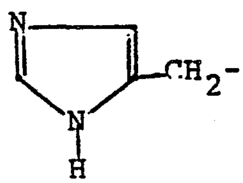
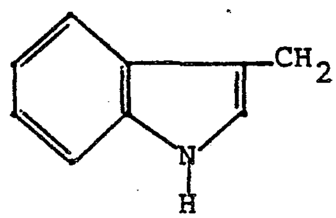
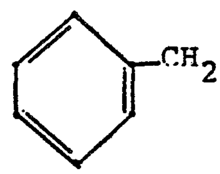

$HO-CH_2-$, $HS-CH_2-$, $H_2N-(CH_2)_4-$, $CH_3-S-(CH_2)_2-$,

$H_2N-(CH_2)_3-$, $H_2N-\overset{NH}{\overset{\|}{C}}-NH-(CH_2)_3-$ and the like.

$R^4$ and $R^5$ when joined through the carbon and nitrogen atoms to which they are attached form a 4- to 6-membered ring which may contain one sulfur atom or a double bond. Preferred rings have the formulae:

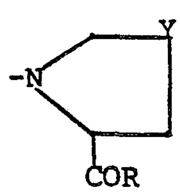

OR

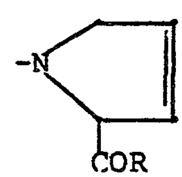

where Y is $CH_2$, S, or $CHOCH_3$.

Preferred are those compounds of Formula (IV) wherein:

$R^6$ can be hydroxy, loweralkoxy, or amino;

R, $R^2$ and $R^7$ are hydrogen;

$R^3$ is loweralkyl, aminoloweralkyl, imidazoyl-loweralkyl, haloloweralkyl;

$R^4$ and $R^5$ are joined to form the preferred rings as defined above where Y is $CH_2$, S, or $CH-OCH_3$;

$R^1$ is as defined previously.

Still more preferred compounds are those preferred compounds of Formula (IV) wherein further $R^1$ is alkyl having from 1 to 8 carbon atoms, substituted lower alkyl wherein the alkvl group has 1-5 carbon atoms and the substituent is amino, arylthio, aryloxy or arylamino, aralkvl or heteroaralkyl wherein the alkyl portion has 1 to 3 carbon atoms such as phenethyl or indolylethyl or substituted arloweralkyl (phenylloweralkyl or naphthylloweralkyl) and substituted heteroarloweralkyl wherein the alkyl groups have 1-3 carbons and wherein the substituent(s) is halo, dihalo, amino, aminoalkyl, hydroxy, loweralkoxy or loweralkyl.

Most preferred are compounds of Formula (IV) wherein

$R^6$ is hydroxy or loweralkoxy;

R, $R^2$ and $R^7$ are hydrogen;

$R^3$ is methyl or aminoloweralkyl;

$R^4$ and $R^5$ are joined through the carbon and nitrogen atom to form proline, 4-thiaproline or 4-methoxy proline;

$R^1$ is alkyl having from 1 to 8 carbon atoms, substituted loweralkyl wherein the alkyl group has 1-5 carbon atoms and the substituent is amino, arylthio or aryloxy, aralkyl or heteroaralkyl wherein the alkyl portion has 1 to 3 carbon atoms such as phenethyl or indolylethyl or substituted aralkyl (phenylloweralkyl or naphthylloweralkyl) and substituted heteroaralkyl wherein the alkyl groups have 1-3 carbons and wherein the substituent(s) is halo, dihalo, amino, aminoalkyl, hydroxy, loweralkoxy or loweralkyl.

The preferred, more preferred and most preferred compounds available under the process of the instant invention are exemplified by:

N-(1(S)-ethoxycarbonyl-3-phenylpropyl)-L-alanyl-L-proline;

N-(1(S)-ethoxycarbonyl-4-methylpentyl)-L-alanyl-L-proline;

N-(1-carboxy-5-aminopentyl)-L-alanyl-L-proline;

N-α-(1(S)-carboxy-3-phenylpropyl)-L-lysyl-L-proline;

N-α-(1(S)-ethoxycarbonyl-3-phenylpropyl)-L-lysyl-L-proline;

N-α-[1(S)-carboxy-3(3-indolyl)propyl]-L-lysyl-L-proline;

N-α-[1(S)-carboxy-3-(4-chlorophenyl)propyl]-L-lysyl-L-proline;

N-α-[1(S)-carboxy-2-phenylthioethyl]-L-lysyl-L-proline;

N-α-[1(S)-carboxy-3-(4-chlorophenyl)propyl]-L-lysyl-L-4α(trans)-methoxyproline;

N-α-[1(S)-carboxy-5-aminopentyl]-L-lysyl-L-proline;
ethyl N-(1(S)-ethoxycarbonyl-3-phenylpropyl)-L-alanyl-L-prolinate;
methyl N-(1-carbomethoxy-3-phenylpropyl)-L-alanyl-L-prolinate (R,S,S) diastereomer;
methyl N-(1-carbomethoxy-3-phenylpropyl)-L-alanyl-L-prolinate (S,S,S) diastereomer;
N-(1-carboxy-3-phenylpropyl)-L-alanyl-L-proline (S,S,S) diastereomer;
N-(1-carboxy-3-phenylpropyl)-L-alanyl-L-proline (R,S,S) diastereomer;
methyl N-(1-carbomethoxy-3-phenylpropyl)-L-lysyl-L-proline;
methyl N-(1-carbomethoxy-3-phenylpropyl)-L-phenylalanyl-L-leucinate;
N-(1-carboxy-3-phenylpropyl)-L-phenylalanyl-L-leucine;
methyl N-(1-carbomethoxy-3-methoxy-3-methylbutyl)-L-alanyl-L-prolinate;
methyl N-(1-carbomethoxy-3-phenyl-trans-2-propenyl)-L-alanyl-L-prolinate;
N-(1-carboxy-3-phenyl-trans-2-propenyl)-L-alanyl-L-proline;
N-(1-carboxy-1-methyl-3-phenylpropyl)-L-alanyl-L-proline;
N-α-(1-carboxy-3-phenylpropyl)-L-ornithyl-L-proline;
N-[1-(ethoxycarbonyl)-3-(4-imidazolyl)propyl]-L-alanyl-L-proline;
N-[1-carboxy-3-(4-imidazolyl)propyl]-L-lysyl-proline; and,
N-(1(S)-carboxy-3-phenylpropyl)-L-alanyl-L-proline;

As will be evident to those skilled in the art and as demonstrated in the Examples, reactive groups not involved in the condensations, such as amino, carboxy, mercapto, etc., may be protected by methods standard in peptide chemistry prior to the coupling reaction and subsequently deprotected to obtain the desired products.

The following reaction schemes (Processes A and B) will aid in the understanding of the instant invention.

PROCESS A

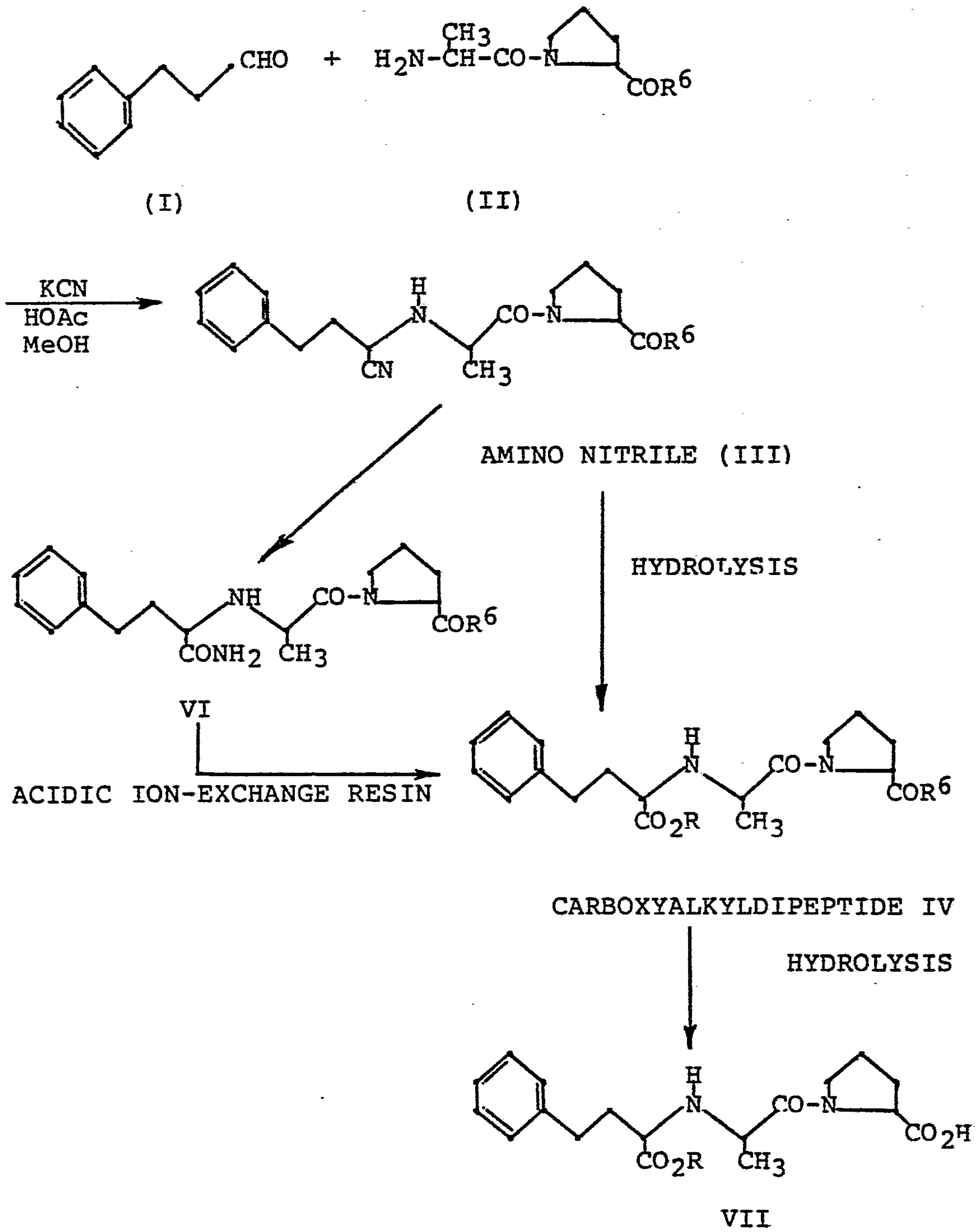
(I)
(II)
KCN
HOAc
MeOH
AMINO NITRILE (III)
HYDROLYSIS
VI
ACIDIC ION-EXCHANGE RESIN
CARBOXYALKYLDIPEPTIDE IV
HYDROLYSIS
VII

In Process A, an aldehyde (or ketone) such as 3-phenylpropionaldehyde (I) is reacted in a solvent such as methanol, with a dipeptide, such as alanylproline (ala-pro) or an esterified dipeptide, and potassium cyanide in the presence of an acid such as acetic acid. The resulting amino nitrile, compound (III), may then be hydrolyzed to yield the amino acid (IV). Other useful solvents in the reaction for the formation of the amino nitrile compound (III) include the lower alkanols, typified by ethanol, propanol, isopropanol, and the like. Generally, the reaction conditions for the formation of (III) are 22°C for about 24 hours. Hydrolysis of compound III to compound (IV) may be accomplished by acid or base hydrolysis, but acid hydrolysis, for example, with anhydrous HCl in methanol, at 0°C for 3-5 hours, is a preferred method. Conversion of compound (IV) to compound (VII) may be accomplished by treating compound (IV) with hydrogen bromide-acetic acid and aqueous hydrobromic acid followed by ion-exchange chromatography and medium pressure liquid chromatography (MPLC) to afford compound (VII).

PROCESS B

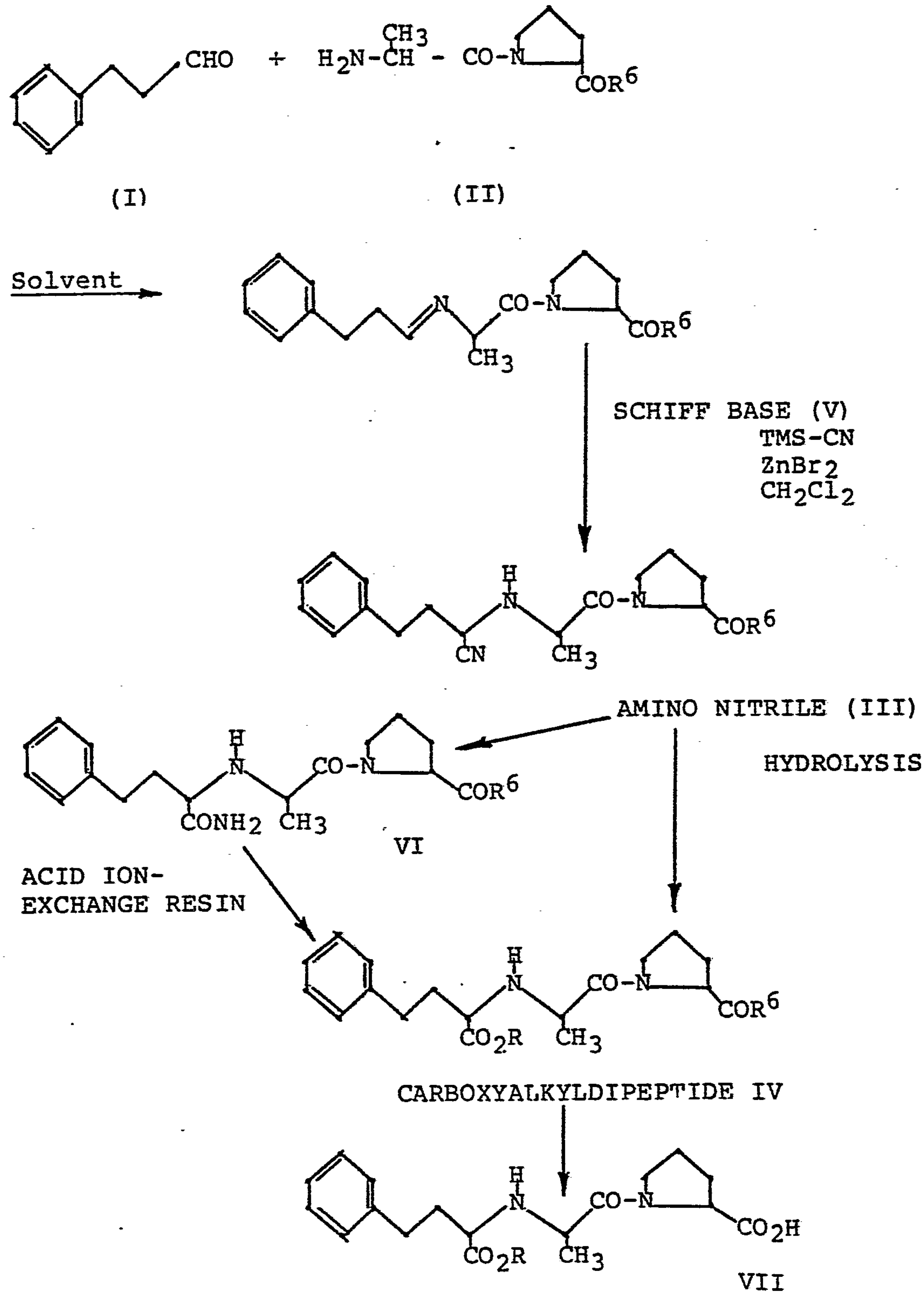
(I)
(II)
Solvent
SCHIFF BASE (V)
TMS-CN
$ZnBr_2$
$CH_2Cl_2$
AMINO NITRILE (III)
HYDROLYSIS
VI
ACID ION-
EXCHANGE RESIN
CARBOXYALKYLDIPEPTIDE IV
VII

In Process B, an aldehyde (or ketone) such as 3-phenylpropionaldehyde (I) is reacted in solution with an esterified dipeptide such as tert-butylalanylprolinate (t-butyl ala-pro). A typical useful reaction solvent is methylene chloride. The resulting Schiff base (V) may be isolated or reacted in situ with trimethylsilylcyanide and zinc bromide to form the amino nitrile compound (III). Hydrolysis of the amino nitrile compound affords the desired carboxyalkyldipeptide (IV).

In addition to the hydrolysis methods described above in Process A, it has been determined that the use of acidic ion-exchange resins provide a mild method for the hydrolytic conversion of unsubstituted carboxamides and carboxylic acid hydrazides to their corresponding acid or ester counterparts.

The hydrolysis of aminonitriles (III) as also described above in Process A, can be used to prepare and isolate carboxamidoalkyl dipeptides (VI) which can subsequently be subjected to the action of acidic ion-exchange resins to provide carboxyalkyldipeptides (IV).

The procedure comprises combining the amide or hydrazide with a large excess by weight (about 15X) of an acidic ion exchange resin such as Amberlyst-15 (Rohm and Haas Co.) in a suitable solvent such as water or a lower alkanol, for example, methanol, ethanol and the like and warming the mixture at reflux. The desired product of the hydrolysis reaction is isolated by elution from the resin using a mixture of the reaction solvent and a

base, for example, pyridine or triethylamine, followed by evaporation of the solvent.

The process of the present invention will best be understood when considered in view of the following examples which further illustrate the practice of Processes A and B for the formation of the carboxyalkyldipeptide compounds of Formula IV.

EXAMPLE 1 (Process A)

Methyl N-(1-carbomethoxy-3-phenyl-1-propyl)-L-alanyl-L-prolinate

To a solution of L-alanyl-L-proline hydrochloride (0.24 g) and 3-phenylpropionaldehyde (0.18 g) in anhydrous methanol (2 ml) was added potassium cyanide (0.088 g). The reaction mixture was tightly stoppered and stirred for 24 hours. Additional methanol (2 ml) was added, the mixture was cooled (ice-bath), and anhydrous hydrogen chloride was passed in until the solution was saturated. The cooled mixture was stirred for 4 hours. After evaporation, the residue was taken up in methylene chloride, filtered, and evaporated. The residue was combined with Amberlyst 15 sulfonic acid resin (Rohm and Haas) (6 g) and methanol (20 ml) and the gently stirred mixture was warmed at reflux for 24 hours. The resin was rinsed with methanol and then the product was eluted with 20:1 $CH_3OH$:pyridine. TLC of the residue after evaporation (silica gel; EtOAc) showed a mixture (1:1) of diastereomers, Rf=0.35, 0.40. Chromatography on silica gel (EtOAc) allowed separation of the diastereomers in a combined yield of 0.23 g (58%).

More-mobile diastereomer (R,S,S):

MS = m/e 376 ($M^+$). NMR ($CDCl_3$): 1.28 (3H, d, J=7); 1.9-2.3 (6H, m); 2.3-2.4 (1H, broad); 2.6-2.8 (2H, m); 3.2-3.3 (1H, m); 3.5-3.6 (2H, m); 3.7-3.8 (1H, m); 3.70 (3H, s); 3.74 (3H, s); 4.5-4.6 (1H, m); 7.1-7.3 (5H, m). IR ($CHCl_3$): 1730, 1640, 1430 $cm^{-1}$.

Less-mobile diastereomer (S,S,S):

MS = m/e 376 ($M^+$). NMR ($CDCl_3$): 1.26 (3H, d, J=7); 1.9-2.3 (6H, m); 2.3-2.4 (1H, broad); 2.6-2.8 (2H, m); 3.20 (1H, t, J=7); 3.4-3.6 (2H, m); 3.7-3.8 (1H, m); 3.66 (3H, s); 3.70 (3H, s); 4.4-4.5 (1H, m); 7.1-7.3 (5H, m). IR ($CHCl_3$): 1730, 1640, 1430 $cm^{-1}$.

## EXAMPLE 2

N-(1-Carboxy-3-phenyl-1-propyl)-L-alanyl-L-proline (S,S,S)

The diastereomer (S,S,S) of methyl N-(1-carbomethoxy-3-phenyl-1-propyl)-L-alanyl-L-prolinate which was less mobile on silica gel in ethyl acetate (50 mg) was combined with 4 equivalents (2.1 ml) of aqueous sodium hydroxide solution (0.25 N). The solution was stirred for 18 hours and then placed onto a column of Dowex 50W-X4 acidic resin (3 g) in $H_2O$. Elution with 20:1 $H_2O$:pyr provided the product, after freeze-drying. Comparisons by TLC, and of NMR and mass spectra showed this material to be identical to that prepared by the method described previously in A. A. Patchett, et al., Nature, 288, 280 (1980).

EXAMPLE 3 (Process A):

Methyl N-(1-carbomethoxy-3-phenyl-1-propyl)-L-lysyl-L-prolinate

To a solution of N-ε-BOC-L-lysyl-L-proline (0.34 g) and 3-phenylpropionaldehyde (0.17 g) in anhydrous methanol (3 ml) there can be added potassium cyanide (0.081 g) and glacial acetic acid (0.060 g). The mixture can be stirred for 24 hours, then diluted with methanol (2 ml), cooled (ice-bath), saturated with anhydrous hydrogen chloride, and the cooled mixture stirred for 24 hours. After evaporation, the residue can be taken up in methanol and filtered. To the filtrate (15 ml) there can then be added Amberlyst 15 sulfonic acid resin (Rohm and Haas) (7.5 g) and the gently-stirred mixture warmed at reflux for 4 days. The resin can then be collected in a small column, rinsed with methanol, and the product eluted with 10:1 $CH_3OH:Et_3N$. Purification can then be achieved by silica gel chromatograpy using $CHCl_3:CH_3OH:H_2O:AcOH$ (85:30:5:1) as eluant.

EXAMPLE 4

N-(1-Carboxy-3-phenyl-1-propyl)-L-lysyl-L-proline

A mixture of methyl N-(1-carbomethoxy-3-phenyl-1-propyl)-L-lysyl-L-prolinate (0.12 g) and 4 equivalents (4.5 ml) of aqueous sodium hydroxide solution (0.25 N) can be stirred for 24 hours and then placed onto a column of Dowex 50W-X4 acidic resin (3 g) in $H_2O$. Elution with 20:1 $H_2O$:pyr and freeze-drying can provide the product. Purification can then be achieved by chromatography on LH-20 (in $CH_3OH$). This material should be identical by TLC, and by NMR and mass spectral analysis to that

prepared by the method described previously in A. A. Patchett, et al., Nature, 288, 280 (1980).

EXAMPLE 5 (Process A):

Methyl N-(1-carbomethoxy-3-phenyl-1-propyl)-L-phenylalanyl-L-leucinate

To a solution of L-phenylalanyl-L-leucine (0.28 g) and 3-phenylpropionaldehyde (0.17 g) in anhydrous methanol (2 ml) was added potassium cyanide (0.081 g) and glacial acetic acid (0.060 g). The mixture was stirred for 48 hours, then diluted with methanol (3 ml), cooled (ice-bath) and saturated with anhydrous hydrogen chloride. The cooled mixture was stirred for 24 hours. The residue after evaporation was taken up in methanol and filtered. To the filtrate (15 ml) was added Amberlyst XN-1010 sulfonic acid resin (Rohm and Haas) (7.5 g) and the gently-stirred mixture was warmed at reflux for 3 days. The resin was collected in a column and washed with methanol. Then the product was eluted with 20:1 $CH_3OH$:pyr. Chromatography on silica gel provided a mixture (1:1) of diastereomers (0.11 g; 0.24 mmol; 24%).

MS: m/e 468 ($M^+$), NMR ($CDCl_3$): 0.9-1.0 (6H, m); 1.5-1.7 (2H, m); 1.7-2.0 (2H, m); 2.18 (1H, m); 2.6-2.8 (2H, m); 3.1-3.5 (4H, m); 3.52, 3.67, 3.68, 3.70 (6H, 4 x s ); 4.5-4.7 (1H, m); 6.9-7.8 (10H, m). IR ($CHCl_3$): 1730, 1640, 1500, 1440 $cm^{-1}$.

EXAMPLE 6

N-(1-carboxy-3-phenyl-1-propyl)-L-phenylalanyl-L-leucine

Using the procedure described above in Example 4, treatment of methyl N-(1-carbomethoxy-3-phenyl-1-propyl)-L-phenylalanyl-L-leucinate with aqueous sodium hydroxide solution can afford the product which can then be purified by ion-exchange chromatography on Dowex 50W-X4.

EXAMPLE 7 (Process A):

Methyl N-(1-carbomethoxy-3-methoxy-3-methyl-1-butyl)-L-alanyl-L-prolinate

To a mixture of L-alanyl-L-proline hydrochloride (0.36 g) and 3,3-dimethylacrolein (0.15 g) in anhydrous methanol (3 ml) was added potassium cyanide (0.11 g). The mixture was stirred for 24 hours, then diluted with methanol (8 ml), cooled (ice-bath), and saturated with anhydrous hydrogen chloride. The reaction vessel was tightly capped and the mixture was allowed to stir for 24 hours at ambient temperature. The residue after evaporation was taken up in methylene chloride and filtered. To a solution of the residue (after evaporation) in methanol (20 ml) was added Amberlyst 15 sulfonic acid resin (Rohm and Haas) (9 g), and the gently-stirred mixture was warmed at reflux for 4 days. The resin was collected in a column and rinsed with methanol. Then the product was eluted with 10:1 $CH_3OH:Et_3N$. TLC on silica gel (20:1 EtOAc:EtOH) showed a mixture (2:1) of diastereomers, Rf=0.40, 0.45. Chromatography on silica gel allowed separation of the diastereomers in a combined yield of 0.32 g (0.89 mmol; 55%).

<u>More-mobile diastereomer</u>: (0.11 g), MS: m/e 358 ($M^+$) NMR ($CDCl_3$) 1.17 (6H, s); 1.20 (3H, d, J=9); 1.8-2.3 (6H, m); 2.7 (1H, broad s); 3.10 (3H, s); 3.2-3.8 (4H, m); 3.63 (6H, s); 4.2-4.4 (1H, m). IR ($CHCl_3$): 1730, 1640, 1430 $cm^{-1}$.

<u>Less-mobile diastereomer</u>: (0.21 g), MS: m/e 358 ($M^+$) NMR ($CDCl_3$) 1.15 (6H, s); 1.22 (3H, d, J=7); 1.8-2.3 (6H, m); 2.9 (1H, broad s); 3.07 (3H, s); 3.2-3.8 (4H, m); 3.73 (6H, s); 4.4-4.6 (1H, m). IR ($CHCl_3$): 1730, 1640, 1430 $cm^{-1}$.

## EXAMPLE 8 (Process B):

<u>Methyl N-(1-carbomethoxy-3-phenyl-trans-2-propenyl)-L-alanyl-L-prolinate</u>

(A) To a solution of cinnamaldehyde (0.132 g) and *t*-butyl-L-alanyl-L-prolinate (0.242 g) in methylene chloride was added freshly-powdered 5A molecular sieves (0.2 g) and silica-alumina catalyst (0.07 g, Aerocat Triple A, American Cyanamid). The mixture was stirred for 5 hours and then trimethylsilylcyanide (0.25 g) and anhydrous zinc bromide (0.01 g) were added. The mixture was stirred for 2 hours, then filtered and concentrated. To the residue there were added ethyl acetate (20 ml) and saturated aqueous ammonium chloride (20 ml) and the two-phase mixture was stirred vigorously for 30 minutes. The organic layer was washed with water and brine and dried ($MgSO_4$). Removal of solvent gave *t*-butyl N-(1-cyano-3-phenyl-trans-2-propenyl)-L-alanyl-L-prolinate (0.36 g). TLC on silica gel (1:1 hexane:EtOAc) showed a mixture (1:1) of diastereomers, Rf=0.25, 0.30.

MS: m/e (383 ($M^+$) NMR ($CDCl_3$): 1.33, 1.37 (3H, 2 x S); 1.42 (9H, s); 1.7-2.1 (4H, m); 2.8 (1H, s); 3.3-3.8 (3H, m); 4.1-4.5 (2H, m): 6.08, 6.11 (1H, 2 x dd, J=6, 16); 6.83 (1H, d, J=16); 7.4 (5H, broad s).

(B) A solution of the material from Example 8(A) (0.26 g) in methanol (3 ml) was cooled (ice-bath) and saturated with anhydrous hydrogen chloride. The solution was stirred at 0°C for 1 hr and then at room temperature for 1 hr. Evaporation provided an oily residue which was taken up in methylene chloride, filtered, and evaporated. The residue was combined with Amberlyst 15 resin (Rohm and Haas) (3.5 g) and methanol (7.5 ml) and the mixture was warmed at reflux for 4 days. The resin was collected in a column and rinsed with methanol. The product was then eluted with 20:1 $CH_3OH$:pyr. Chromatography on silica gel (EtOAc) provided the product (52 mg, 21%) as a mixture (1:1) of diastereomers, Rf=0.30, 0.35. MS: m/e 374 ($M^+$). NMR ($CDCl_3$): 1.27, 1.30 (3H, 2 x d, J=7); 1.6-2.2 (4H, m); 2.4-2.6 (1H, broad); 3.4-3.7 (3H, m); 3.67, 3.70 (3H, 2 x s): 3.95 (1H, d, J=8); 4.3-4.7 (1H, m); 6.10 (1H, dd, J=8, 15); 6.63 (1H, d, J=15); 7.4 (5H, broad s).

## EXAMPLE 9

N-(1-Carboxy-3-phenyl-trans-2-propenyl)-L-alanyl-L-proline

A mixture of the above diester (Examle 8B) (25 mg) and aqueous sodium hydroxide (1N; 1 ml) was stirred for 4 hours and then placed onto a column of Dowex 50W-X4 resin (4 g) in water. The product was

eluted with 20:1 $H_2O$:pyr and freeze-dried. TLC (silica gel; EtOAc, n-BuOH HOAc, $H_2O$ 3:1:1:1), Rf=0.30. MS m/e 473 (($M^+$ bis trimethylsilyl-15). NMR (DMSO-$d_6$): 1.16, 1.18 (3H, 2 x d, J=4); 1.4-1.9 (4H, m); 2.0-2.2 (1H, broad); 3.2-4.4 (4H, m); 6.09, 6.12 (1H, 2 x dd, J=5.16); 6.61, 6.68 (1H, 2 x d, J=5); 7.2-7.5 (6H, m).

## EXAMPLE 10

N-(1-Carboxy-1-methyl-3-phenyl-1-propyl)-L-alanyl-L-proline

(A) To a solution of 4-phenyl-2-butanone (0.19 g) and t-butyl-L-alanyl-L-prolinate (0.28 g) in methylene chloride (3 ml) was added freshly-powdered 5A molecular sieves (0.22 g) and silica-alumina catalyst (0.07 g, Aerocat Triple A-American Cyanamid). The mixture was stirred under nitrogen for 48 hours, and then trimethylsilylcyanide (0.29 g) and anhydrous zinc bromide (0.01 g) were added. The mixture was stirred for 4 hours, then filtered and evaporated. To the residue was added ethyl acetate (20 ml) and saturated aqueous ammonium chloride (20 ml) and the two-phase mixture was stirred vigorously for 30 minutes. The organic layer was washed with $H_2O$ and brine and dried ($MgSO_4$). Evaporation gave t-butyl N-(1-cyano-1-methyl-3-phenyl-1-propyl)-L-alanyl-L-prolinate (0.36 g). TLC on silica gel (hexanes:EtOAc, 1:1) showed a mixture (1:1) of diastereomers, Rf=0.40, 0.45. MS: m/e 399 ($M^+$), 327 ($M^+$-HCN) NMR ($CDCl_3$): 1.28 (3H, d, J=7); 1.42 (9H, s); 1.8-2.2 (6H, m); 2.05 (3H, s); 2.7-3.0 (4H, m); 3.4-3.8 (2H, m); 4.3-4.5 (1H, m); 7.1 (5H, broad s).

(B) A solution of the material from Example 10(A) (0.10 g) in concentrated aqueous hydrochloric acid (10 ml) was cooled, and anhydous hydrogen chloride was bubbled in slowly for 30 minutes. The solution was tightly capped and allowed to stir at room temperature for 24 hours. The residue afer evaporation was combined with Amberlyst 15 resin (1.5 g, Rohm and Haas) and methanol (5 ml) and the gently stirred mixture was warmed at reflux for 3 days. The resin was collected in a column and rinsed with methanol. Then the product was eluted with 10:1 $CH_3OH$:$Et_3N$. TLC on silic gel (EtOAc) showed a mixture (1:1) of diastereomers, Rf=0.35, 0.40. Purification was achieved by chromatography on silica gel. MS: m/e 390 ($M^+$).

(C) A mixture of the material from Example 10(B) (16 mg) and aqueous sodium hydroxide solution (0.25 N; 0.60 ml) was stirred for 24 hours under nitrogen, then placed onto a column of Dowex 50W-X4 (3 g) in $H_2O$. The product was eluted with 20:1 $H_2O$:pyr and freeze-dried to a white foam. TLC on silica gel (EtOAc, n-BuOH, $H_2O$, HOAc, 3:1:1:1), Rf=0.5. MS: m/e 491 ($M^+$ bistrimethylsilyl-15).

## EXAMPLE 11

N-(1-Carboxamido-3-phenyl-1-propyl)-L-alanyl-L-proline

L-Alanyl-L-proline hydrochloride (1.25 mmol, 0.275 g) was combined with 3-phenylpropionaldehyde (1.54 mmol, 0.206 g) and potassium cyanide (1.54 mmol; 0.100 g) in anhydrous methanol (2 ml). After 24 hours, solvent was removed and the residue taken up in concentrated hydrochloric acid (5 ml). After 24 hours, water (10 ml) was added and the solution was

extracted with $CH_2Cl_2$ (3 x 5 ml). The solution was then evaporated to a white foam. Purification on DOWEX 50W-X4 resin (with 50:1 $H_2O$:pyridine as eluant) followed by silica gel chromatography (85:30:5:1 $CHCl_3$:$CH_3OH$:$H_2O$:HOAc) provided the product as a solid. TLC (85:30:5:1 CMWA) Rf=0.30, 0.35. NMR ($D_2O$): 1.55, 1.61 (3H, 2 x d, J=7); 1.9-2.1 (2H, m); 2.2-2.4 (4H, m); 2.7-2.9 (2H, m); 3.5-3.7 (2H, m); 3.8-4.0 (1H, m); 4.1-4.4 (1H, m); 7.3-7.5 (5H, m); MS: m/e 476 ($M^+$, bis-trimethyl-silyl-15).

## EXAMPLE 12

Methyl N-(1-Carboxamido-3-phenyl-1-propyl)-L-alanyl-L-prolinate

L-Alanyl-L-proline hydrochloride (1.00 mmol; 0.222 g), 3-phenylpropionaldehyde (1.25 mmol; 0.168 g), and potassium cyanide (1.25 mmol; 0.081 g) were combined with anhydrous methanol (2 ml). The mixture was stirred for 24 hours, diluted with methanol 5 ml), cooled (ice-bath) and saturated with anhydrous hydrogen chloride. Water (0.03 ml) was added and the cooled mixture was stirred for 3 hours. The mixture was evaporated to a white foam which was taken up in $CH_2Cl_2$ and filtered. The filtrate was then saturated with anhydrous ammonia and immediately concentrated to dryness. The residue was slurried in ethyl acetate and filtered through celite. The residue, after evaporation, was purified by chromatography on silica gel (2:1 hexane:EtOAc). Initial fractions contained methyl N-(1-carbomethoxy-3-phenyl-1-propyl)-L-alanyl-L-prolinate (0.138 g; 0.368 mmol; 37%). The amidoester was then collected (0.185 g; 0.514 mmol; 51%) as a colorless oil. TLC (silica gel; EtOAc; 2

developments) Rf-0.10, 0.15. NMR ($CDCl_3$): 1.28, 1.45 (3H, 2 x d, J=7); 1.9-2.5 (6H, m); 2.6-3.0 (2H, m); 3.5-4.0 (3H, m); 3.68 (3H, s); 4.5-4.7 (1H, m); 5.6-5.8 (1H, broad); 6.3-6.5 (1H, broad); 7.1-7.3 (5H, m). MS: m/e 317 ($M^+$ $-CONH_2$).

## EXAMPLE 13

Methyl N-(1-carbomethoxy-3-phenyl-1-propyl)-L-alanyl-L-prolinate

Treatment of the amidoacid prepared as described in Example 11 or of the amidoester prepared as described in Example 12 with a 15-fold excess (by weight) of Amberlyst 15 acidic resin in methanol for 48 hours at reflux can provide methyl N-(1-carbomethoxy-3-phenyl-1-propyl)-L-alanyl-L-prolinate as described in Example 1. Separation of the amidoesters by silica gel chromatography followed by treatment of each with Amberlyst 15 acidic resin can allow isolation of each diastereomer of the diester.

## EXAMPLE 14

When ethanol is substituted for methanol in the Amberlyst 15 resin treatment in Example 1, the diester ethyl N-(1-carboethoxy-3-phenyl-1-propyl)-L-alanyl-L-prolinate can be isolated. Separation of the diastereomers can be accomplished by silica gel chromatography.

## EXAMPLE 15

Treatment of the diastereomer (of the diester described in Example 14) which is less mobile on silica gel (in hexanes - EtOAc), with a 4:1 mixture of concentrated HBr in HOAc and concentrated aqueous

HBr can provide N-(1-(S)-carboethoxy-3-phenyl-1-propyl)-L-alanyl-L-proline, which can then be converted to its maleate salt.

Alternatively, treatment of the mixture of diastereomers of the diester described in Example 14, can provide the same product after purification and separation of diastereomers by reverse-phase HPLC or crystallization of the maleate salt.

EXAMPLE 16

When L-alanyl-L-proline amide hydrochloride is substituted for L-alanyl-L-proline hydrochloride in Example 12, the diamide, N-(1-carboxamido-3-phenyl-1-propyl)-L-alanyl-L-proline amide, can be isolated as a mixture (1:1) of diastereomers. Separation of the diastereomers can be achieved by chromatography on silica gel.

EXAMPLE 17

Treatment of each diastereomer of the diamide of Example 16 with a 15-fold excess (by weight) of Amberlyst 15 acidic resin in methanol can provide the corresponding diastereomer diester, N-(1-carbomethoxy-3-phenyl-1-propyl)-L-alanyl-L-prolinate.

WHAT IS CLAIMED IS:

1. A process for preparing compounds of the Formula (IV):

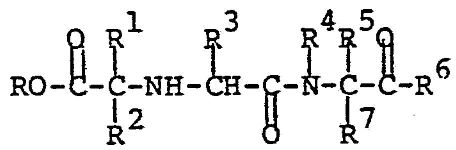

(IV)

wherein

$R^6$ is hvdroxy, loweralkoxy, or amino;

R, $R^2$ and $R^7$ are hydrogen or loweralkyl;

$R^3$ is hydrogen,
loweralkyl,
phenylloweralkyl,
aminomethylphenylloweralkyl,
hydroxyphenylloweralkyl,
hydroxyloweralkyl,
acetylaminoloweralkyl,
acylaminoloweralkyl,
aminoloweralkvl;

$R^1$ is hydrogen,
alkyl of from 1 to 20 carbon atoms, including branched, cyclic and unsaturated alkyl groups,
substituted loweralkyl wherein the substitutent is halo,
hydroxy,
loweralkoxy,
aryloxy,
amino,
loweralkylamino,
diloweralkylamino,
acylamino,

arylamino,
guanidino,
imidazolyl,
indolyl,
mercapto,
loweralkylthio,
arylthio,
carboxy,
carboloweralkoxy,
dimethylaminoloweralkyl,
haloloweralkyl,
mercaptoloweralkyl and
loweralkylthioloweralkyl;

$R^4$ is hydrogen or
loweralkyl,

$R^5$ is hydrogen,
loweralkyl,
phenyl,
phenylloweralkyl,
hydroxyphenylloweralkyl,
hydroxyloweralkyl,
aminoloweralkyl,
mercaptoloweralkyl or
loweralkylthioloweralkyl; and,

$R^4$ and $R^5$ may be connected together to form an alkylene bridge of from 2 to 4 carbon atoms, an alkylene bridge of from 2 to 4 carbon atoms containing a double bond or an alkylene bridge as above, substituted with

hydroxy,
loweralkoxy or
loweralkyl;

which comprises reacting a compound of the Formula (I):

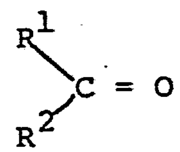

(I)

wherein $R^1$ and $R^2$ are as defined above, with a dipeptide (or protected dipeptide) of the Formula (II):

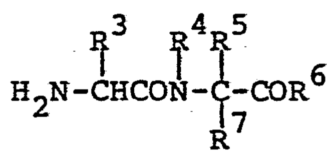

(II)

wherein $R^3$, $R^4$, $R^5$, $R^6$ and $R^7$ are as defined above and may include suitable protection of any of the reactive groups $R^3$, $R^4$, $R^5$, $R^6$ or $R^7$;

and a cyanide compound selected from the alkali metal cyanides or trimethylsilylcyanide, to form an amino nitrile compound of the Formula (III):

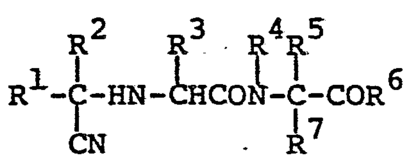

(III)

followed by hydrolysis.

2. The process of Claim 1 wherein said Formula (IV) compound is:

N-(1-ethoxycarbonyl-3-phenylpropyl)-L-alanyl-L-proline;

N-(1-ethoxycarbonyl)-4-methylpentyl-L-alanyl-L-proline;

N-(1-carboxy-5-aminopentyl)-L-alanyl-L-proline;

N-α-(1-ethoxycarbonyl-3-phenylpropyl)-L-lysyl-L-proline;

Methyl N-(1-carbomethoxy-3-phenylpropyl)-L-alanyl-L-prolinate (R,S,S) diastereomer;

Methyl N-(1-carbomethoxy-3-phenylpropyl)-L-alanyl-L-prolinate (S,S,S) diastereomer;

N-(1-carboxy-3-phenylpropyl)-L-alanyl-L-proline (S,S,S) diastereomer;

N-(1-carboxy-3-phenylpropyl)-L-alanyl-L-proline (R,S,S) diastereomer;

Methyl N-(1-carbomethoxy-3-phenylpropyl)-L-lysyl-L-proline;

N-(1-carboxy-3-phenylpropyl)-L-lysyl-L-proline (R, S, S) diastereomer;

N-(1-carboxy-3-phenylpropyl)-L-lysyl-L-proline (S,S,S) diastereomer;

Methyl N-(1-carbomethoxy-3-phenylpropyl)-L-phenylalanyl-L-leucinate;

N-(1-carboxy-3-phenylpropyl)-L-phenylalanyl-L-leucine;

Methyl N-(1-carbomethoxy-3-methoxy-3-methylbutyl)-L-alanyl-L-prolinate;

Methyl N-(1-carbomethoxy-3-phenyl-trans-2-propenyl)-L-alanyl-L-prolinate;

N-(1-carboxy-3-phenyl-trans-2-propenyl)-L-alanyl-L-proline;

N-(1-carboxy-1-methyl-3-phenylpropyl)-L-alanyl-L-proline;

N-α-[1-carboxy-3-(3-indolyl)propyl]-L-lysyl-L-proline,

N-α-[1-carboxy-3-(4-chlorophenyl)propyl]-L-lysyl-L-proline,

N-α-[1-carboxy-2-phenylthioethyl]-L-lysyl-L-proline,

N-α-[1-carboxy-3-(4-chlorophenyl)propyl]-L-lysyl-trans-4-methoxy-L-proline,

N-α-[1-carboxy-5-aminopentyl]-L-lysyl-L-proline,

N-α-(1-carboxy-3-phenylpropyl)-L-ornithyl-L-proline,

N-[1-ethoxycarbonyl)-3-(4-imidazolyl)propyl]-L-alanyl-L-proline,

N-[1-carboxy-3-(4-imidazolyl)propyl]-L-lysyl-L-proline,

N-(1(S)-carboxy-3-phenylpropyl)-L-alanyl-L-proline, or

Ethyl N-(1(S)-ethoxycarbonyl-3-phenylpropyl)-L-alanyl-L-prolinate.

3. The process of Claim 1 wherein the alkali metal cyanide compound is potassium cyanide.

4. The process of Claim 1 wherein the cyanide compound is trimethylsilyl cyanide.

5. The process of Claim 1 wherein the dipeptide (II) has the Formula:

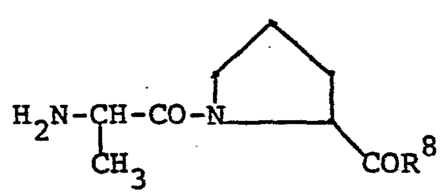

wherein $R^8$ is hydroxy, loweralkoxy, or amino.

6. The process of Claim 1 wherein the hydrolysis of the amino nitrile compound (III) is accomplished using about a 15 times excess (by weight) of an acidic ion exchange resin warmed to reflux in a solvent selected from water or a lower alkanol.

7. The process of Claim 1 wherein the hydrolysis of the amino nitrile compound is acid hydrolysis using a mineral acid in a lower alkanol.

8. The process of Claim 7 wherein the mineral acid is HCl and the lower alkanol is methanol.

9. The process of Claim 7 wherein the mineral acid is HCl and the lower alkanol is ethanol.